# EUROPEAN PATENT APPLICATION

(11) **EP 2 937 332 A1**
(43) Date of publication of application: **28.10.2015**
(21) Application number: 14382144.5
(22) Date of filing: 22.04.2014
(51) Int. Cl.: C07C 231/02, C07B 43/06, C07C 233/18

(54) **Process for preparing a solid hydroxyalkylamide**

(71) Applicant: Cromogenia Units, S.A., 08040 Barcelona (ES)
(72) Inventor: Torrelles Nogués, Antonio, 08040 Barcelona (ES); Morell García, Albert, 08040 Barcelona (ES)
(74) Representative: Oficina Ponti, SLP

(57) **Abstract**

The present invention relates to a process for preparing a solid hydroxyalkylamide comprising the steps of: (a) reacting an alkyl ester of a dicarboxylic acid with an alkanolamine in the presence of a basic catalyst at a temperature in the range between 80°C and 100°C until no ester is detected in the reaction mixture; (b) adding a mixture of water and acid; and (c) solidifying the product obtained in step (b).

## Description

### FIELD OF INVENTION

The present invention relates to a process for preparing a solid hydroxyalkylamide which is useful as an acid polyester-based curing agent for powder coating as an alternative to TGIC (Triglycidylisocyanurate).

### BACKGROUND Of THE INVENTION

Powder coating compositions allow to avoid the use of organic solvents in liquid paints. This is advantageously important since organic solvents used in liquid coatings tend to volatilize. In order to make powder coatings triglycidylisocyanurate (TGIC) and some other chemicals like hydroxylamides with two or more hydroxyl groups are used. They are useful as a crosslinker, particularly the N,N,N',N'-tetrakis(2-hydroxyethyl)adipamide, or the bis-(N,N'-di-ethylhydroxy)adipoyl amide or bis-diethanoladipamide.

Some hydroxyalkylamides (in particular, the bis-diethanoladipamide) have been synthesized as a crosslinker by reaction of dimethylesters with alkanolamines, like diethanolamine. This kind of products are obtained by recrystallizing, usually in methanol/acetone, cooling for growing the crystals, filtering and drying (see, for example, J. Coat. Tech., 50 (643), 49-55 (1978); US-A-4,032,460; US-A-4,076,917; US-A-4,493,909; US-A-4,727,111 and Japanese Patent 56-062895) or by prilling/flaking.

Direct flaking is not a valid option because in a molten state after some time (from some minutes until one hour as maximum) and depending basically on parameters, such as the temperature and the amount and type of catalyst, the hydroxyalkylamides, like bis-diethanoladipamide, degrade to low melting point mixtures, giving liquid products even at room temperature, which makes them unsuitable for use in powder coatings, since they are thermodynamically unstable.

The recrystallization process is typically performed by dissolving the crude hydroxyalkylamide (Bis-Diethanoladipamide) in a solvent, such as methanol/acetone, cooling the solution to grow the crystals, filtering off the product from the mother liquors, and drying the product until no residual solvent is present.

With prilling/flaking processes, the hydroxyalkylamide (Bis-Diethanoladipamide) must be maintained in a molten state for several hours until the operation is complete. During this period of time some of the product in the molten state degrades to undesirable by-products.

Another problem associated with the hydroxyalkylamides (Bis-Diethanoladipamide) prepared by these known processes is that they are generally soft, sticky, waxy solids, which typically render the materials unsuitable for use in powder coatings, which particularly require free-flowing powders.

In addition, for a large scale preparation, due to the thermodynamic characteristics, this kind of products are not stable in molten state. In fact, beta-hydroxyalkylamides, like bis-diethanoladipamide, must be dissolved in an organic solvent, like methanol, or even mixtures like methanol/acetone, and proceed in production by recrystallization with cooling, or by solvent evaporation at relatively low temperature. No engines are available for flaking big amounts of this compound in a very short time, without loosing quality for the application of this solid in powder coatings.

The present invention provides a process for preparing a solid hydroxyalkylamide, in particular, bis-diethanoladipamide, overcoming the above mentioned problems associated with the known processes in the art.

### SUMMARY OF THE INVENTION

The present invention relates to a process for preparing a solid hydroxyalkylamide comprising the steps of:
(a) reacting an alkyl ester of a dicarboxylic acid with an alkanolamine in the presence of a basic catalyst at a temperature in the range between 80°C and 100°C until no ester is detected in the reaction mixture;
(b) adding a mixture of water and acid;
(c) solidifying the product obtained in step (b).

In particular, the alkyl ester of a dicarboxylic acid is dimethyladipate, the alkanolamine is 2-diethanolamine and consequently the hydroxyalkylamide is bis-diethanoladipamide.

### DETAILED DESCRIPTION OF THE INVENTION

The present invention relates to a process for preparing a solid hydroxyalkylamide comprising the steps of:
(a) reacting an alkyl ester of a dicarboxylic acid with an alkanolamine in the presence of a basic catalyst at a temperature in the range between 80°C and 100°C until no ester is detected in the reaction mixture;
(b) adding a mixture of water and acid;
(c) solidifying the product obtained in step (b).

In a preferred embodiment, the process disclosed herein further comprises the step of: (d) drying the solidified product obtained in step (c).

In a further preferred embodiment, the product after step (c) and after optional step (d) has around 1% wt of water present in said product. This represents a molar ratio between the hydroxyalkylamide and water of around 6. Nevertheless, the amount of water present in the solid product (dried or not dried) depends on the final application and even for some applications the molar ratio between the hydroxyalkylamide and water can be around 2 (about 3% wt of water present in the solidified product)

In another preferred embodiment of the process disclosed herein including any of the previous embodiments, said temperature in step (a) is in the range between 80°C and 85°C.

In still another preferred embodiment of the process disclosed herein including any of the previous embodiments, the amount of water added in step (b) is between 1 and 3 wt% with respect of the total composition.

In still another preferred embodiment of the process disclosed herein including any of the previous embodiments, said basic catalyst is selected from potassium methoxide, potassium tert-butoxide and a mixture thereof.

In still another preferred embodiment of the process disclosed herein including any of the previous embodiments, the amount of catalyst is in the range from 0.1 to 1.0 wt% based on the weight of the ester of step (a).

In still another preferred embodiment of the process disclosed herein including any of the previous embodiments, the solidification step (c) is carried out by a method selected from flaking, extrusion and countercurrent-spray drying.

In still another preferred embodiment of the process disclosed herein including any of the previous embodiments, the acid in step (c) is an organic carboxylic acid, such as formic acid, acetic acid, lactic acid, preferably acetic acid, or a mineral acid, preferably phosphoric acid.

In still another preferred embodiment of the process disclosed herein including any of the previous embodiments, the alkyl ester of dicarboxylic acid in step (a) is at least one alkyl ester of a C2-C12 dicarboxylic acid, preferably is at least one selected from dimethyladipate, dimethylglutarate and a mixture thereof.

In a more preferred embodiment of the process disclosed herein including any of the previous embodiments, the alkyl ester of a dicarboxylic acid is dimethyladipate, the alkanolamine is 2-diethanolamine and the hydroxyalkylamide is bis-diethanoladipamide. For this preferred embodiment, the reactions are shown in the following scheme:

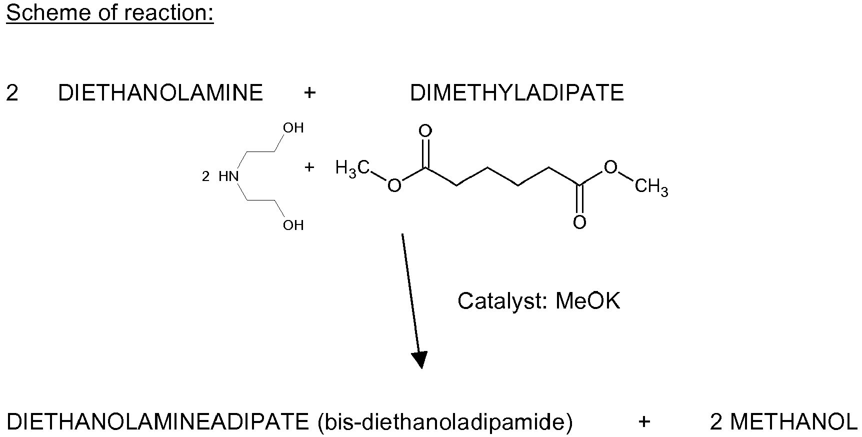

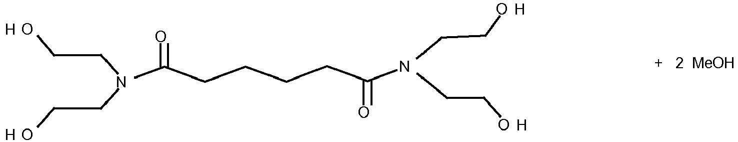

It is important to note that the temperature of the crude reaction mixture is controlled until the hydroxyalkylamide (preferably, Bis-Diethanoladipamide) becomes a white coloured wax. It is recommendable to keep the product under stirring, but without melting the entire product. The range of temperatures in the reaction mixture is maintained between 80°C and 100°C, preferably between 80-85°C in order to maintain the product in a pasty form, without reaching the liquid state. At the same time, the alcohol obtained as by-product is distilled.

Once the addition of reactants of step (a) is complete, samples are taken continuously and the ester band is analyzed by any suitable technique, for example, infrared spectroscopy (IR) until no ester is detected. Obviously this will also depend on the technique being used. If the technique is not very precise, there will be more probabilities of having traces of ester in the reaction mixture.

At this point water and acid, preferably acetic acid, are added, which leave the reaction mixture with a moisture content of 3% maximum with respect of the total composition, and without catalyst because it has been neutralized by the acid.

The obtained pasty product is usually maintained for about 4 to 5 hours and finally is solidified by a suitable method including, without being limited thereto, flaking, extrusion and countercurrent-spray drying methods.

The optional additional drying process can be performed by a vacuum drying system with stirring and crushing. At this time, the residual alcohol obtained as a by-product, preferably methanol if the alkyl ester of dicarboxylic acid is dimethyladipate, the alkanolamine is 2-diethanolamine and the hydroxyalkylamide is bis-diethanoladipamide, is usually removed at atmospheric or reduced pressure.

The obtained solid is a hydroxyalkylamide, preferably, bis-diethanoladipamide, having the consistency of a free-flowing powder suitable for the direct use as a crosslinker in powder coating formulations.

The solid hydroxyalkylamides, preferably, bis-diethanoladipamide, produced by the process disclosed herein have been improved compared to products produced by prior art methods of recrystallization because no solvents are involved in the final stages, no additional steps of solvent removal/recovery are necessary, and because of the improved characteristics (e.g. free-flowing powder) of the hydroxyalkylamides, preferably, bis-diethanoladipamide, which are at least equal or exceed those obtained from recrystallization.

In addition, the hydroxyalkylamide, preferably, bis-diethanoladipamide, obtained with the process disclosed herein is a product of much higher overall quality (i.e. fewer impurities are present) because said process does not subject the hydroxyalkylamides, preferably, bis-diethanoladipamide, to long hold times at elevated temperatures in the molten state during which times undesirable by-products are formed. Also, the operation of filtration, usually associated with recrystallization routes, is not necessary in the process disclosed herein.

The present invention will now be described by way of examples which do not intend to limit the scope of the instant invention.

### EXAMPLES

### EXAMPLE 1 (General process)

Diethanolamine was loaded into a glass reactor and dried under vacuum over 110°C until the water content was below 0.1 %. Once the diethanolamine was dried, and under constant nitrogen sparging, it was heated to 80-85°C and a first addition of potassium methoxide was carried out, 33% of the total amount. After the incorporation of one third of the total amount of the catalyst, the addition of dimethyladipate started and it took about 2 hours.One third of the total amount of the catalyst was added after 40 minutes and after 80 minutes since the dimethyladipate addition started .The catalyst was therefore added in 3 additions. During the addition of dimethyladipate, methanol was distilled. Once the dimethyladipate was completely added, a control of the ester present in the reaction mixture was performed by Infrared spectroscopy (IR) constantly, until the ester band (1700 cm-1) disappeared. At this moment water was quickly added to stop the reaction, along with acetic acid to neutralize the catalyst. After the addition, the product passed from a liquid state to a white solid form with a melting point of about 125°C. If stirring was required, the temperature could be increased to 95-100°C to keep the product pasty, but under stirring.

It was very important to control the reaction continuously using infrared spectroscopy (IR), because within few minutes the product could degrade again giving esters, rising the amine value and lowering the melting point. Therefore, IR monitoring was required to follow how the reaction was taking place.

### EXAMPLE 2

First it was checked that the reactor was clean and dry, and that the raw materials (dimethyladipate and diethanolamine) did not contain any moisture by Karl Fisher method. The humidity had to be less than 0.1 % wt. Diethanolamine had to be dried under vacuum in the presence of N₂ at 120-130°C to completely eliminate the moisture, until the value by the Karl Fisher method was <0.1 %. The water and acetic acid were also prepared. The preparation of 250 kg of dietanolamineadipate for a pilot plant was as follows:
Once the diethanolamine (DEA) was dried, the dried 156.5 kg of the diethanolamine in the reactor were heated between 80 and 85°C. Then 1.08 kg of catalyst (potassium methoxide) was charged, and the addition of 129.7 kg of dimethyladipate (DMAd) started and took about 2 hours. 1.08 kg catalyst was added after 40 minutes and after 80 minutes since the dimethyladipate addition started. The whole process had to be carried out under constant nitrogen sparging. During the reaction, methanol was distilled (approximately 47.7 kg), and had to be remove to keep warm the pipe to the condenser, so that methanol did not return to the reaction mixture. As the reaction took place the product became a white coloured wax. It was important to keep the product under stirring, but without melting the entire product. If necessary, the temperature was raised from 85°C to 100°C, in order to maintain the product pasty, not in liquid form. Once the addition was completed, samples were continuously taken and the ester band was analyzed by infrared spectroscopy (IR) until it disappeared or became minimum, and the amide band became maximum. At this point water and acetic acid were added, leaving the product with a moisture content of 3%, and without catalyst because it was neutralized by acetic acid giving sodium acetate and methanol. After the reaction for up to 4 to 5 hours the product was flaked for solidification and preserved. Subsequently, by using a vacuum drying system with stirring and crushing (type Lödigue) moisture was removed by the evaporation of water and the residual methanol, to obtain the desired final product. During the drying process 12.5 kg .of methanol and 7.5 kg of water were evaporated.

Experimental conditions and analytical data obtained from on-line sampling are presented in Table 1.

**TABLE 1**

| Time (min) | Temperature (°C) | Operation |
|---|---|---|
| 0 | | DEA charged and add 1/3 catalyst and start adding DMAd |
| 49.00 | 84.20 | Add 1/3 catalyst |
| 80.00 | 84.60 | Distillation begins very slowly |
| 87.00 | 87.30 | The temperature is raised to 88-90°C |
| 103.00 | 88.90 | Add 1/3 catalyst |
| 158.00 | 88.40 | Addition of DMAd is complete |
| 193.00 | 85.10 | Pasty white product |
| 205.00 | 88.30 | Adding acetic acid for the catalyst neutralization |
| 210.00 | 88.10 | Adding remaining water |

| | | |
|---|---|---|
| DEA: 2-diethanolamine DMAd: dimethyladipate | | |

**TABLE 2**

| Physical Appearance of final product | White solid |
|---|---|
| % residual DEA (titration with HClO4) | 2% |
| M.P. (°C) (DSC) | 124°C |

## Claims

1. Process for preparing a solid hydroxyalkylamide comprising the steps of:
(a) reacting an alkyl ester of a dicarboxylic acid with an alkanolamine in the presence of a basic catalyst at a temperature in the range between 80°C and 100°C until no ester is detected in the reaction mixture;
(b) adding a mixture of water and acid;
(c) solidifying the product obtained in step (b).

2. Process according to claim 1, further comprising the step of:
(d) drying the solidified product obtained in step (c).

3. Process according to any of the preceding claims, wherein the final amount of water is in a molar relationship between hydroxyalkylamide and water of 2 to 6.

4. Process according to any of the preceding claims, wherein said temperature is in the range between 80°C and 85°C.

5. Process according to any of the preceding claims, wherein the amount of water added in step (b) is between 1 and 3 wt% with respect of the total composition.

6. Process according to any of the preceding claims, wherein said basic catalyst is selected from potassium methoxide, potassium tert-butoxide and a mixture thereof.

7. Process according to any of the preceding claims, wherein the amount of catalyst is in the range from 0.1 to 1.0 wt% based on the weight of the ester of step (a).

8. Process according to any of the preceding claims, wherein the solidification step (c) is carried out by a method selected from flaking, extrusion and countercurrent-spray drying.

9. Process according to any of the preceding claims, wherein said acid in step (c) is an organic carboxylic acid, or a mineral acid.

10. Process according to any of the preceding claims, wherein the alkyl ester of a dicarboxylic acid is at least one alkyl ester of a C2-C12 dicarboxylic acid.

11. Process according to claim 10, wherein the alkyl ester of a dicarboxylic acid is at least one selected from dimethyladipate, dimethylglutarate and a mixture thereof.

12. Process according to any of the preceding claims, wherein the alkanolamine is selected from diethanolamine, monoethanolamine and diisopropanolamine.

13. Process according to any of the preceding claims, wherein the alkyl ester of dicarboxylic acid is dimethyladipate, the alkanolamine is 2-diethanolamine and the hydroxyalkylamide is bis-diethanoladipamide.
